# EUROPÄISCHE PATENTSCHRIFT

(11) **EP 2 036 527 B1**
(45) Veröffentlichungstag und Bekanntmachung des Hinweises auf die Patenterteilung: **27.10.2010**
(21) Anmeldenummer: 08016144.1
(22) Anmeldetag: 12.09.2008
(51) Int. Cl.: A61H 35/04

(54) **Nasendusche mit Rückschlagventil**
Nasal spray with non-return valve
Douche nasale dotée d'un clapet anti-retour

(30) Priorität: 13.09.2007 DE 202007012817 U
(43) Veröffentlichungstag der Anmeldung: 18.03.2009
(73) Patentinhaber: Siemens & Co. Heilwasser und Quellenprodukte des Staatsbades Bad Ems GmbH & Co. KG, 56130 Bad Ems (DE)
(72) Erfinder: Hirsch, Olaf, 56076 Koblenz (DE)
(74) Vertreter: Philipp, Matthias

(56) Entgegenhaltungen:
- EP-A- 1 304 097
- DE-A1- 1 500 177
- FR-A- 2 297 032
- FR-A- 2 864 448
- US-A- 2 052 321
- US-A- 2 485 184

## Beschreibung

Die Erfindung betrifft eine Nasendusche mit einem elastischen Behälter und einem Rückschlagventil.

Nasenduschen sind in unterschiedlichsten Ausführungsformen bekannt, wobei in der Regel ein abnehmbarer Deckel vorgesehen ist, in dem eine Belüftungsöffnung angeordnet ist, die zum Gebrauch entweder offengelassen wird, wobei dann die in dem Behälter aufgenommene Spülflüssigkeit allein aufgrund ihrer Schwerkraft ausläuft, oder bedarfsweise mit einem Finger verschlossen werden kann, so daß durch Druck auf den elastischen Behälter die Spülflüssigkeit unter Überdruck herausgedrückt werden kann.

Die Aufgabe der vorliegenden Erfindung besteht darin, die aus FR-A-2864448 bekannte Nasendusche dahingehend weiter zu verbessern, daß sowohl unter Normaldruck als auch unter Überdruck bei einfachster Handhabung gearbeitet werden kann.

Diese Aufgabe wird durch eine Nasendusche mit einem elastischen Behälter zur Aufnahme von Spülflüssigkeit, der eine Spülöffnung und eine mit einem Finger verschließbare Belüftungsöffnung aufweist, gelöst, wobei sich die Nasendusche dadurch auszeichnet, daß die Belüftungsöffnung mit einem Rückschlagventil versehen ist, das im Gebrauch bei drucklosem Behälter geöffnet ist und ein Austreten von Spülflüssigkeit aus der Spülöffnung zuläßt, und das durch rasches Zusammendrücken des Behälters geschlossen werden kann und ein Austreten von Spülflüssigkeit unter Druck aus der Spülöffnung ermöglicht.

Weiterhin sieht die Erfindung vor, daß das Rückschlagventil aufweist:
- einen Verschlußkörper (6), der mit einem Führungsabschnitt (20) in einer Längsführung (8) zwischen einer Öffnungsstellung und einer Schließstellung beweglich geführt ist,
- eine an einem ersten Ende der Längsführung (8) angeordnete erste ringförmige Sitzfläche (12), die in der Öffnungsstellung mit einer an einem ersten Ende des Verschlußkörpers (6) ausgebildeten ersten ringförmigen Dichtfläche (22) abdichtend zusammenwirkt,
- eine an einem zweiten Ende der Längsführung (8) angeordnete zweite ringförmige Sitzfläche (14), die in der Schließstellung mit einer an einem zweiten Ende des Verschlußkörpers (6) ausgebildeten zweiten ringförmigen Dichtfläche (24) abdichtend zusammenwirkt,
- und einen innerhalb des Führungsabschnitts (20) angeordneten Durchgang (30), der mit einer ersten Öffnung (32) an dem ersten Ende des Verschlußkörpers (6) innerhalb der ersten Dichtfläche (22) und mit einer zweiten Öffnung (34) in einem Bereich zwischen der ersten und zweiten Dichtfläche (22, 24) des Führungsabschnitts (20) nach außen mündet,
- wobei die Anordnung so ist, daß der Verschlußkörper (6) in der Öffnungsstellung eine Strömung durch die erste Öffnung (32) und entlang der zweiten Sitzfläche (14) zuläßt und in der Schließstellung eine Strömung entlang der zweiten Sitzfläche (14) verhindert.

Die Erfindung sieht bevorzugt vor, daß die Belüftungsöffnung bei geöffnetem Rückschlagventil mit einem Finger verschließbar ist.

Das Rückschlagventil kann einen beweglichen Verschlußkörper aufweisen, der beispielsweise kegel- oder kugelförmig sein kann.

Die Belüftungsöffnung kann an einem Ende eines Belüftungskanals ausgebildet sein, der eine Erweiterung zum Aufnehmen des Verschlußkörpers aufweist.

Es kann vorgesehen sein, daß die Längsführung hohlzylindrisch ist. Entsprechend kann vorgesehen sein, daß der Führungsabschnitt zylindrisch ist.

Es ist zweckmäßig, wenn die Längsführung einen größeren Durchmesser als der Führungsabschnitt aufweist.

Der Durchgang kann entlang einer Längsachse des Führungsabschnitts angeordnet sein.

Bei zylindrischer Ausbildung von Führungsabschnitt oder Längsführung kann vorgesehen sein, daß ein Abstand zwischen Öffnungs- und Schließstellung des Verschlußkörpers etwa 5 % bis 50 % des Durchmessers des Führungsabschnitts oder des Durchmessers der Längsführung oder 5% bis 50% der Länge des Führungsabschnitts oder der Länge der Längsführung beträgt.

Es ist zweckmäßig, wenn der Verschlußkörper mit Ausnahme eines Teils des Durchgangs rotationssymmetrisch ist.

Der Führungsabschnitt kann in einem Bereich, in dem der Durchgang zwischen der ersten und zweiten Dichtfläche mündet, eine oder mehrere Abflachungen aufweisen.

Es kann vorgesehen sein, daß die Längsführung in einem Deckel, insbesondere Schraub- oder Klemmdeckel, für einen Behälter ausgebildet ist.

Weitere Vorteile und Merkmale der Erfindung ergeben sich aus der nachfolgenden Beschreibung eines Ausführungsbeispiels, wobei auf eine Zeichnung Bezug genommen ist, in der
Fig. 1 einen Querschnitt durch einen Schraubdeckel mit einem darin angeordneten Rückschlagventil zeigt,
Fig. 2 eine Seitenansicht des Verschlußkörpers des in Fig. 1 dargestellten Rückschlagventils zeigt,
Fig. 3 eine Schnittansicht des Verschlußkörpers entlang Linie III - III in Fig. 2 zeigt, und
Fig. 4 eine Nasendusche zeigt, die mit einem Rückschlagventil nach Fig. 1 bis 3 versehen ist.

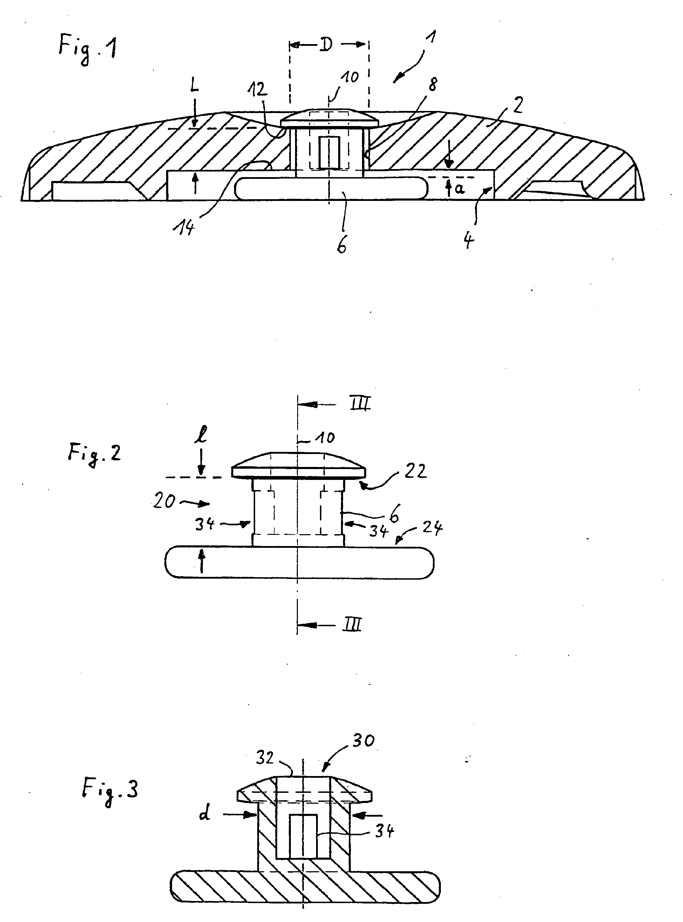

Fig. 1 erläutert ein Rückschlagventil 1, das beispielhaft in einem Schraubdeckel 2 für eine Nasendusche angeordnet ist. Der Deckel 2 ist mit einem nicht im einzelnen dargestellten Innengewinde 4 versehen und kann auf einen entsprechenden Schraubstutzen der in Fig. 1 ebenfalls nicht dargestellten Nasendusche aufgeschraubt werden.

Das Rückschlagventil besteht in dem hier dargestellten Beispiel aus einem Verschlußkörper 6, der in einer Längsführung 8 entlang einer Längsachse 10 längsverschieblich geführt ist. Die Längsführung 8 ist durch eine zylindrische Öffnung in dem Deckel 2 gebildet und weist an einem ersten Ende eine erste ringförmige Sitzfläche 12 und an einem gegenüberliegenden zweiten Ende eine ringförmige zweite Sitzfläche 14 auf. In dem Bereich zwischen den Sitzflächen 12, 14 hat die Längsführung 8 einen Durchmesser D und eine Länge L (Abstand der Sitzflächen 12, 14 entlang der Längsachse 10 gesehen).

Der Verschlußkörper 6 weist einen im wesentlichen zylindrischen Führungsabschnitt 20 auf, mit dem er mit Spiel innerhalb der Längsführung 8 längsverschieblich geführt ist, wobei ein Außendurchmesser d des Führungsabschnitts 20 kleiner ist als der Innendurchmesser D der Längsführung 8.

Der Führungsabschnitt 20 ist an beiden Enden mit einer Verdickung versehen, die einen größeren Durchmesser als die Längsführung 8 aufweist, wobei an einem ersten Ende des Ventilkörpers eine erste ringförmige Dichtfläche 22 ausgebildet ist, die in der in Fig. 1 dargestellten Öffnungsstellung des Rückschlagventils mit der ersten Sitzfläche 12 der Längsführung 8 abdichtend zusammenwirkt. An einem gegenüberliegenden zweiten Ende des Führungsabschnitts 20 ist der Verschlußkörper mit einer zweiten Dichtfläche 24 versehen, die in einer gegenüber der in Fig. 1 dargestellten Öffnungsstellung nach oben verlagerten Schließstellung des Verschlußkörpers mit der zweiten Sitzfläche 14 der Längsführung 8 abdichtend zusammenwirkt. Der Abstand der Dichtflächen 22, 24 legt eine Länge 1 des Führungsabschnitts fest.

Der Verschlußkörper ist, wie aus Fig. 1 ersichtlich, zwischen der dargestellten Öffnungsstellung und der nach oben verlagerten Schließstellung um einen Abstand a verlagerbar, der in einer bevorzugten Ausführung zwischen etwa 5 % und 50 % des Durchmessers d oder D betragen kann, oder aber zwischen 5% und 50% der Länge 1 oder L.

Der Verschlußkörper 6 ist weiterhin mit einem Durchgang 30 versehen, der im wesentlichen zylindrisch ausgebildet ist und entlang der Längsachse 10 verläuft. Der Durchgang mündet im Bereich des ersten Endes des Führungsabschnitts 20 innerhalb der ersten Dichtfläche 22 in einer ersten Öffnung 32 nach außen, wobei die erste Öffnung 32 von der ersten Dichtfläche 22 umschlossen ist und konzentrisch innerhalb dieser angeordnet ist.

Der Durchgang 30 erstreckt sich ausgehend von der ersten Öffnung 32 entlang der Längsachse 10 in Richtung auf das zweite Ende des Führungsabschnitts 20, allerdings nicht bis zu der zweiten Dichtfläche 24 oder über diese hinaus, sondern endet noch in einem Bereich innerhalb der zweiten Dichtfläche 24, oder von der ersten Öffnung 32 aus gesehen vor der zweiten Dichtfläche, in einer zweiten Öffnung 34 in einer radial nach außen weisenden Richtung.

Fig. 2 und 3 erläutern weiter die Ausbildung des Durchgangs 30. Wie Fig. 3 zeigt, besteht dieser in der hier dargestellten Ausführungsform, ausgehend von der ersten Öffnung 32, zunächst aus einer zylindrischen Sacköffnung, in deren von der ersten Öffnung 32 abgekehrten unterem Endabschnitt ein radial auswärts verlaufender Abschnitt und die zweite Öffnung 34 ausgebildet sind. In dem hier gezeigten Beispiel sind zwei derartige Abschnitte und Öffnungen 34 mit jeweils rechteckigem Querschnitt ausgebildet, die um 180° versetzt bzw. einander gegenüberliegend angeordnet sind.

Wie man Fig. 1 entnimmt, braucht sich die zweite Öffnung 34 bzw. brauchen sich die zweiten Öffnungen 34 nicht bis in den Bereich der zweiten Dichtfläche 24 zu erstrecken, um in der Öffnungsstellung einen Strömungsdurchgang an der zweiten Dichtfläche vorbei zu ermöglichen, da der Führungsabschnitt 20 mit Spiel in der Längsführung 8 aufgenommen ist, wodurch ein radialer Freiraum gebildet ist, durch den eine Strömungsverbindung zwischen der zweiten Öffnung 34 und dem Bereich der zweiten Dichtfläche 24 bzw. einem Bereich zwischen der zweiten Dichtfläche 24 und zweiten Sitzfläche 14 gebildet ist.

In einer anderen Ausführung, bei der die Längsführung 8 einen Innendurchmesser hat, der im wesentlichen dem Außendurchmesser des Führungsabschnitts 20 entspricht, mündet die zweite Öffnung 34 etwa in Höhe der zweiten Dichtfläche 24, damit in der Öffnungsstellung eine Strömungsverbindung zwischen der ersten Öffnung 32 des Durchgangs 30 und dem Bereich zwischen zweiter Dichtfläche 24 und zweiter Sitzfläche 14 möglich ist.

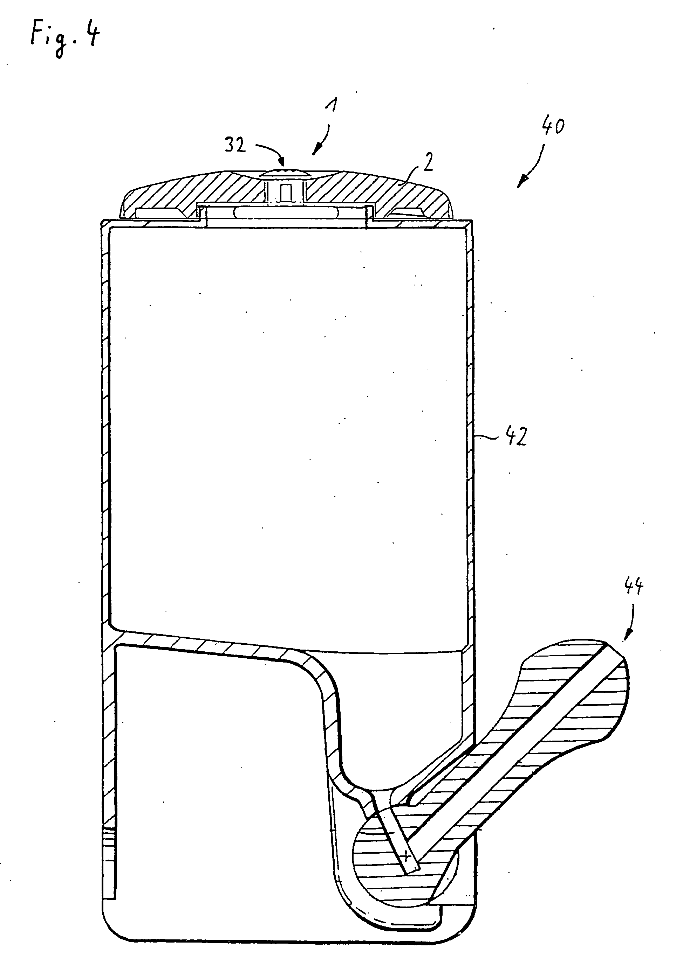

Fig. 4 zeigt eine Schnittansicht einer im Ganzen mit 40 bezeichneten Nasendusche, die mit einem Behälter 42, einer am Ende eines Schwenkventils angeordneten Spülöffnung 44 und einem in einem Deckel 2 angeordneten Rückschlagventil 1 gemäß Fig. 1 bis 3 versehen ist.

In der Öffnungsstellung des Rückschlagventils kann Spülflüssigkeit drucklos aus der Spülöffnung auslaufen und bedarfsweise durch Verschließen der Belüftungsöffnung (erste Öffnung 32) mit einem Finger am Auslaufen gehindert werden. Umgekehrt kann durch einen raschen Druck auf den Behälter das Rückschlagventil geschlossen werden, so daß anschließend ein Spülen mit Überdruck möglich ist, ohne daß die Öffnung 32 verschlossen gehalten werden muß.

In einer alternativen Ausführungsform, in der das Rückschlagventil nicht entsprechend Fig. 1 bis 3 ausgebildet ist, kann das Rückschlagventil einen beweglichen Verschlußkörper in Form beispielsweise einer Kugel oder eines Kegels aufweisen, der in einem Aufnahmeraum lose aufgenommen ist, der als Erweiterung im Verlauf eines Belüftungskanals ausgebildet ist. Innerhalb des Aufnahmeraums kann sich der Verschlußkörper in Längsrichtung des Belüftungskanals zwischen einer ersten und einer zweiten Endstellung bewegen. Die erste, bezüglich der Nasendusche nach außen weisende Endstellung legt eine Verschlußstellung fest, in der der Verschlußkörper abdichtend mit einer Sitzfläche zusammenwirkt, die ringförmig um den Belüftungskanal herum ausgebildet ist. In der zweiten, entgegengesetzten Endstellung dichtet der Verschlußkörper nicht mit einer Dichtfläche ab, sondern läßt eine Strömung von außen in die Nasendusche hinein zu. Dies kann beispielsweise dadurch realisiert werden, daß der Verschlußkörper auf einer ins Innere der Nasendusche weisenden Seite keine glatte Anlagefläche, sondern vorstehende Rippen oder Stege aufweist, oder daß umgekehrt die zum Inneren der Nasendusche hin angeordnete Anlagefläche für den Verschlußkörper nicht eben, sondern mit Rippen, Stegen o.ä. ausgebildet ist, so daß eine dichtende Anlage des Verschlußkörpers in dieser Endstellung nicht möglich ist und Luft nach innen strömen kann.

### Bezugszeichenliste

- 1: Rückschlagventil
- 2: Deckel
- 4: Innengewinde
- 6: Verschlußkörper
- 8: Längsführung
- 10: Längsachse
- 12: erste Sitzfläche
- 14: zweite Sitzfläche
- 20: Führungsabschnitt
- 22: erste Dichtfläche
- 24: zweite Dichtfläche
- 30: Durchgang
- 32: erste Öffnung
- 34: zweite Öffnung
- 40: Nasendusche
- 42: Behälter
- 44: Spülöffnung

- a: Abstand
- d: Durchmesser von 20
- D: Durchmesser von 8
- 1: Länge von 20
- L: Länge von 8

## Patentansprüche

1. Nasendusche (40) mit einem elastischen Behälter (42) zur Aufnahme von Spülflüssigkeit, der eine Spülöffnung (44) und eine mit einem Finger verschließbare Belüftungsöffnung (32) aufweist, die mit einem Rückschlagventil (1) versehen ist, das im Gebrauch bei drucklosem Behälter (42) geöffnet ist und ein Austreten von Spülflüssigkeit aus der Spülöffnung (44) zuläßt, und das durch rasches Zusammendrücken des Behälters (42) geschlossen werden kann und ein Austreten von Spülflüssigkeit unter Druck aus der Spülöffnung (44) ermöglicht, **dadurch gekennzeichnet, daß** das Rückschlagventil (1) folgende Merkmale aufweist:
- einen Verschlußkörper (6), der mit einem Führungsabschnitt (20) in einer Längsführung (8) zwischen einer Öffnungsstellung und einer Schließstellung beweglich geführt ist,
- eine an einem ersten Ende der Längsführung (8) angeordnete erste ringförmige Sitzfläche (12), die in der Öffnungsstellung mit einer an einem ersten Ende des Verschlußkörpers (6) ausgebildeten ersten ringförmigen Dichtfläche (22) abdichtend zusammenwirkt,
- eine an einem zweiten Ende der Längsführung (8) angeordnete zweite ringförmige Sitzfläche (14), die in der Schließstellung mit einer an einem zweiten Ende des Verschlußkörpers (6) ausgebildeten zweiten ringförmigen Dichtfläche (24) abdichtend zusammenwirkt,
- und einen innerhalb des Führungsabschnitts (20) angeordneten Durchgang (30), der mit einer die Belüftungsöffnung bildenden ersten Öffnung (32) an dem ersten Ende des Verschlußkörpers (6) innerhalb der ersten Dichtfläche (22) und mit einer zweiten Öffnung (34) in einem Bereich zwischen der ersten und zweiten Dichtfläche (22, 24) des Führungsabschnitts (20) nach außen mündet,
- wobei die Anordnung so ist, daß der Verschlußkörper (6) in der Öffnungsstellung eine Strömung durch die erste Öffnung (32) und entlang der zweiten Sitzfläche (14) zuläßt und in der Schließstellung eine Strömung entlang der zweiten Sitzfläche (14) verhindert.

2. Nasendusche nach Anspruch 1, **dadurch gekennzeichnet, daß** die Längsführung (8) hohlzylindrisch ist.

3. Nasendusche nach Anspruch 1 oder 2, **dadurch gekennzeichnet, daß** der Führungsabschnitt (20) zylindrisch ist.

4. Nasendusche nach einem der vorangehenden Ansprüche, **dadurch gekennzeichnet, daß** die Längsführung (8) einen größeren Durchmesser (D) als der Führungsabschnitt (20) aufweist.

5. Nasendusche nach einem der vorangehenden Ansprüche, **dadurch gekennzeichnet, daß** der Durchgang (30) entlang einer Längsachse (10) des Führungsabschnitts (20) angeordnet ist.

6. Nasendusche nach einem der Ansprüche 2 bis 5, **dadurch gekennzeichnet, daß** ein Abstand (a) zwischen Öffnungs- und Schließstellung des Verschlußkörpers (6) etwa 5 % bis 50 % des Durchmessers (d) des Führungsabschnitts (20) oder des Durchmessers (D) der Längsführung (8) oder 5% bis 50% der Länge (1) des Führungsabschnitts (20) oder der Länge (L) der Längsführung (8) beträgt.

7. Nasenduschel nach einem der vorangehenden Ansprüche, **dadurch gekennzeichnet, daß** der Verschlußkörper (6) mit Ausnahme eines Teils des Durchgangs (30) rotatiönssymmetrisch ist.

8. Nasendusche nach einem der vorangehenden Ansprüche, **dadurch gekennzeichnet, daß** der Führungsabschnitt (20) in einem Bereich, in dem der Durchgang (30) zwischen der ersten und zweiten Dichtfläche (22, 24) mündet, eine oder mehrere Abflachungen aufweist.

9. Nasendusche nach einem der vorangehenden Ansprüche, **dadurch gekennzeichnet, daß** die Längsführung (8) in einem Deckel, insbesondere Schraub- oder Klemmdeckel, für einen Behälter ausgebildet ist.

## Claims

1. Nasal spray (40) with a flexible container (42) to hold rinsing fluid, which has a rinsing opening (44) and a ventilation hole (32), which can be closed off with a finger and is provided with a non-return valve (1), which is opened in use when the container (42) is unpressurised and allows rinsing fluid to pass out of the rinsing opening (44), and which can be closed by rapidly compressing the container (42) and enables rinsing fluid to pass out of the rinsing opening (44) under pressure, **characterised in that** the non-return valve (1) has the following features:
- a closure body (6) which is movably guided with a guide section (20) in a longitudinal guide (8) between an opening position and a closure position,
- a first ring-shaped seat surface (12), arranged on a first end of the longitudinal guide (8), which in the opening position co-operates with a first ring-shaped sealing surface (22) formed at a first end of the sealing body (6) to form a seal,
- a second ring-shaped seat surface (14), arranged on a second end of the longitudinal guide (8), which in the closed position co-operates with a second ring-shaped sealing surface (24) formed at a second end of the sealing body (6) to form a seal,
- and a passage (30) arranged within the guide section (20) which opens outwards with a first opening (32) forming the ventilation hole at the first end of the closure body (6) within the first sealing surface (22) and with a second opening (34) in an area between the first and second sealing surfaces (22, 24) of the guide section (20),
- whereby the arrangement is such that in the open position, the closure body (6) allows a flow through the first opening (32) and along the second seat surface (14) and in the closed position prevents any flow along the second seat surface (14).

2. Nasal spray according to claim 1, **characterised in that** the longitudinal guide (8) is hollow-cylindrical.

3. Nasal spray according to claim 1 or 2, **characterised in that** the guide section (20) is cylindrical.

4. Nasal spray according to one of the preceding claims, **characterised in that** the longitudinal guide (8) has a larger diameter (D) than the guide section (20).

5. Nasal spray according to one of the preceding claims, **characterised in that** the passage (30) is arranged along a longitudinal axis (10) of the guide section (20).

6. Nasal spray according to one of claims 2 to 5, **characterised in that** a distance (a) between opening and closure position of the closure body (6) amounts to some 5% to 50% of the diameter (d) of the guide section (20) or of the diameter (D) of the longitudinal guide (8) or 5% to 50% of the length (1) of the guide section (20) or of the length (L) of the longitudinal guide (8).

7. Nasal spray according to one of the preceding claims, **characterised in that** the closure body (6) is rotationally symmetrical with the exception of part of the passage (30).

8. Nasal spray according to one of the preceding claims, **characterised in that** the guide section (20) has one or more flattened areas in an area in which the passage (30) opens between the first and second sealing surface (22, 24).

9. Nasal spray according to one of the preceding claims, **characterised in that** the longitudinal guide (8) is designed as part of a lid, in particular a screw or clamping lid, for a container.

## Revendications

1. Douche nasale (40) avec un récipient (42) élastique, pour contenir du liquide de rinçage, présentant une ouverture de rinçage (44) et une ouverture d'aération (32), obturable avec un doigt, dotée d'un clapet anti-retour (1) qui, en utilisation, lorsque le récipient (42) sans pression, est ouvert et permet une sortie de liquide de rinçage hors de l'ouverture de rinçage (44), et qui peut être fermée par une rapide compression du récipient (42) et permet une sortie de liquide de rinçage sous pression, hors de l'ouverture de rinçage (44), **caractérisée en ce que** le clapet anti-retour (1) présente les caractéristiques suivantes :
- un corps de fermeture (6), guidé de manière déplaçable, avec un tronçon de guidage (20), dans un guidage longitudinale, entre une position d'ouverture et une position de fermeture,
- une première surface de siège (12) en forme d'anneau, disposée à une première extrémité du guidage longitudinal (8), coopérant en produisant une étanchéité, dans la position d'ouverture, avec une première surface d'étanchéité (22) en forme d'anneau, réalisée sur une première extrémité du corps de fermeture (6),
- une deuxième surface de siège (14) en forme d'anneau, disposée à une deuxième extrémité du guidage longitudinal (8), coopérant en produisant une étanchéité, dans la position de fermeture, avec une deuxième surface d'étanchéité (24) en forme d'anneau, réalisée sur une deuxième extrémité du corps de fermeture (6),
- et un passage (30), disposé à l'intérieur du tronçon de guidage (20), débouchant vers l'extérieur, par une première ouverture (32) formant l'ouverture d'aération, sur la première extrémité du corps de fermeture (6), à l'intérieur de la première surface de guidage (22), et, par une deuxième ouverture (34), dans une zone située entre la première et la deuxième surface d'étanchéité (22, 24) du tronçon de guidage (20),
- l'agencement étant tel que, dans la position d'ouverture, le corps de fermeture (6) permet un écoulement à travers la première ouverture (32) et le long de la deuxième surface de guidage (14) et, dans la position de fermeture, empêche un écoulement le long de la deuxième surface de siège (14).

2. Douche nasale selon la revendication 1, **caractérisée en ce que** le guidage longitudinal (8) est cylindrique creux.

3. Douche nasale selon la revendication 1 ou 2, **caractérisée en ce que** le tronçon de guidage (20) est cylindrique.

4. Douche nasale selon l'une des revendications précédentes, **caractérisée en ce que** le guidage longitudinale (8) présente un diamètre (D) plus gros que celui du tronçon de guidage (20).

5. Douche nasale selon l'une des revendications précédentes, **caractérisée en ce que** le passage (30) est disposé le long d'un axe longitudinal (10) du tronçon de guidage (20).

6. Douche nasale selon l'une des revendications 2 à 5, **caractérisée en ce qu'**un espacement (a), entre position d'ouverture et de fermeture du corps de fermeture (6), est d'à peu près 5 % à 50 % du diamètre (d) du tronçon de guidage (20) ou du diamètre (D) du guidage longitudinal (8), ou de 5 % à 50 % de la longueur (1) du tronçon de guidage (20) ou de la longueur (L) du guidage longitudinal (8).

7. Douche nasale selon l'une des revendications précédentes, **caractérisée en ce que** le corps de fermeture (6) répond à une symétrie de rotation, à l'exception d'une parti du passage (30).

8. Douche nasale selon l'une des revendications précédentes, **caractérisée en ce que**, dans une zone, dans laquelle le passage (30) débouche entre la première et la deuxième surface d'étanchéité (22, 24), le tronçon de guidage (20) présente un ou plusieurs méplats.

9. Douche nasale selon l'une des revendications précédentes, **caractérisée en ce que** le guidage longitudinale (8) est réalisé dans un couvercle, en particulier un couvercle vissé ou serré, pour un récipient.
